# NEUE EUROPÄISCHE PATENTSCHRIFT

(11) **EP 3 067 025 B2**
(45) Veröffentlichungstag und Bekanntmachung des Hinweises auf die Entscheidung über den Einspruch: **14.07.2021**
(45) Hinweis auf die Patenterteilung: 05.09.2018
(21) Anmeldenummer: 16000412.3
(22) Anmeldetag: 18.02.2016
(51) Int. Cl.: A61F 13/20

(54) **VERFAHREN UND VORRICHTUNG ZUM HERSTELLEN EINES TAMPONS SOWIE DAMIT HERGESTELLTER TAMPON**
METHOD AND APPARATUS FOR MAKING A TAMPON AND TAMPON MADE BY THIS
PROCEDE ET DISPOSITIF DE FABRICATION D'UN TAMPON ET TAMPON AINSI FABRIQUE

(30) Priorität: 11.03.2015 DE 202015001935 U
(43) Veröffentlichungstag der Anmeldung: 14.09.2016
(73) Patentinhaber: Rauscher Consumer Products GmbH, 1140 Wien (AT)
(72) Erfinder: PÖTSCH, Ernst, 2525 Schönau / Triesting (AT); SCHIERBAUER, Claudia, 1190 Wien (AT)
(74) Vertreter: Seranski, Klaus

(56) Entgegenhaltungen:
- EP-A1- 2 431 014
- EP-A1- 2 740 448
- EP-A1- 2 749 261
- WO-A1-94/15564
- DE-U1-202012 011 679
- US-A1- 2003 135 180
- US-B2- 8 827 974

## Beschreibung

Die Erfindung betrifft ein Verfahren zum Herstellen eines in eine Körperhöhle, insbesondere in die weibliche Scheide, einführbaren Tampons mit einem im Wesentlichen 5 zylinderförmigen Saugkörper, der eine sich in Richtung auf ein axiales Ende des Saugkörpers verjüngende Kuppe mit einem konvexen Scheiteibereich aufweist, bei dem eine Materialbahn aus saugfähigem Material zu einem Wickel aufgewickelt und der Wickel zumindest abschnittweise in ***10*** radialer und/oder axialer Richtung verpreßt wird.

Dabei bezeichnet der Ausdruck "konvexer Scheitelbereich" im Rahmen der Beschreibung und Ansprüche eine Oberflächenform, bei der einzelne Punkte auf der Oberfläche mit Hilfe von innerhalb der Kuppe verlaufenden Linien miteinander verbunden werden können. Dadurch unterscheidet sich eine Kuppe mit einem konvexen Scheitelbereich von einer Kuppe mit einer Vertiefung im Scheltelbereich, wie sie beispielsweise in der EP 2 740 448 A1 beschrieben ist.

Tampons werden schon seit langer Zeit zur Aufnahme von Körperflüssigkeit in der Wundversorgung, maßgeblich aber im Zusammenhang mit der weiblichen Menstruation, eingesetzt. Ein einfacher Tampon, bestehend aus einem Pfropf aus einem aufsaugfähigem Material, einer Umhüllung aus Gaze, einem Rückholfaden und einem Trichter aus durchlässigem Material, ist bereits in der 1936 vom Reichspatentamt herausgegebenen Patentschrift 624 395 beschrieben. Die Entwicklung entsprechender Tampons ist bis heute nicht abgeschlossen. Beispielhaft wird für die ***30*** einzelnen Entwicklungsschritte Bezug genommen auf die deutsche Patentschrift 804 835 aus dem Jahr 1949, die deutsche Offenlegungsschrift 27 22 802 A1 aus dem Jahr 1977, die deutsche Patentanmeldung 35 19 515 A1 aus dem Jahr 1985, die internationale Patentanmeldung mit der ***35*** Veröffentlichungsnummer WO 01/24729 A2 aus dem Jahr 2001, die europäische Patentanmeldung mit der Veröffentlichungsnummer 2 298 259 A aus dem Jahr 2010 und die danach veröffentlichte europäische Patentanmeldung 2 431 014 A1.

Schon In der Patentschrift 624 395 wird darauf hingewiesen, daß eine Umkleidung des Saugkörpers erforderlich ist, um zu verhindern, daß Rückstände des faserigen Saugkörpers in der Körperhöhle verbleiben. In der DE 35 19 515 A1 wird ein Verfahren vorgeschlagen, wie ein ***45*** zur Umhüllung des Saugkörpers dienendes Hüllmaterial auf einen gewickelten Tampon aufgebracht werden kann. Bei dem in dieser Schrift beschriebenen Verfahren wird ein Vliesstoffabschnitt auf einem Watteband fixiert, das Watteband aufgewickelt, das hintere Vliesstoffende auf dem ***so*** darunterliegenden Vliesstoffmaterial festgelegt und das so erhaltene Vorprodukt verpreßt, um so einerseits eine zuverlässige, dem Heraustreten von Fasern aus der Watte entgegenwirkende Umhüllung bereitzustellen und andererseits eine Ausdehnung des Saugkörpers bei Flüssigkeitseintritt ohne Behinderung durch den Vliesstoff zu ermöglichen. Bei den so hergestellten Tampons wird dem "Ausfusseln" des Saugkörpers mit Hilfe des Vliesstoffmaterials entgegengewirkt. Zur Vermeidung vom Behinderungen bei der Kuppenformung erstreckt sich das Vliesstoffmaterial bei Verfahren der in der DE 35 19 516 A1 beschriebenen Art üblicherweise nur über einen Teil der Wattebandbreite, so daß die Kuppe nicht mehr von dem Vliesstoffmaterial abgedeckt wird. Ferner hat es sich gezeigt, daß das Saugvermögen der so erhaltenen Tampons in vielen Fällen nicht vollständig ausgeschöpft werden kann und Flüssigkeit aus der Körperhöhle austritt, obwohl der Saugkern des Saugkörpers noch nicht mit Flüssigkeit gesättigt bzw, vollständig genutzt ist. Diese Mängel konnten auch nicht durch Bereitstellung von sich parallel zur Wickelachse erstreckenden Rillen in der äußeren Begrenzungsfläche des Saugkörpers vollständig beseitigt werden.

Eine Lösung der angesprochenen Probleme wird in der EP 2 298 259 A angeboten. Der in dieser Schrift beschriebene Tampon weist im Scheitelbereich der das Einführende des Tampons bildenden Kuppe eine Vertiefung auf, durch welche die dort aufgegriffene Körperflüssigkeit direkt in den Saugkern des Saugkörpers gelangt, so daß eine frühzeitige Sättigung des äußeren Bereichs des Saugkörpers im Kuppenbereich zuverlässig verhindert werden kann und auch der innere Bereich des Tampons optimal genutzt wird.

Eine ähnliche Lösung wird in der EP 2 431 014 A1 vorgeschlagen. Bei einer ersten Ausführungsform der in dieser Schrift beschriebenen Tampons wird ein mit einem Hüllvlies ausgestattetes Watteband aufgewickelt und dann axial und radial derart verpreßt, daß an einem Ende des so hergestellten Tampons eine mit einer Vertiefung ausgestattete Kuppe entsteht.

Bei einer zweiten Ausführungsform von in der genannten Schrift beschriebenen Tampons wird ein mit einem Hüllvlies beidseits abgedeckter Wattebandstreifen W-förmig gefaltet und radial sowie axial verpreßt. Dadurch wird ein Tampon erhalten, dessen äußere Begrenzungsfläche vollständig von dem Hüllvlies abgedeckt ist. Die Herstellung eines solchen Tampons durch Faltung eines Wattebandabschnitts erweist sich allerdings als problematisch.

Endlich wird bei einer dritten Ausführungsform der in der genannten Schrift beschriebenen Erfindung ein erster Wattebandabschnitt kreuzförmig von einem zweiten Wattebandabschnitt überlagert. Die freiliegenden Endbereiche der Wattebandabschnitte werden hochgeklappt und das so erhaltene kastenförmige Gebilde wird radial und axial verpreßt. Auch dieses Herstellungsverfahren erweist sich als problematisch.

In der EP 2 740 448 und in der DE 20 2012 011 679 U1 wird eine Weiterbildung der bekannten Tampons mit einer Vertiefung im Bereich der Kuppe vorgeschlagen, bei der eine etwa senkrecht zur Wickelachse verlaufende Stirnfläche des Wickels vor der Verpressung vollständig mit einem flüssigkeitsdurchlässigen Hüllmaterial abgedeckt wird. Beim Einsatz der in dieser Schrift beschriebenen Tampons hat es sich als problematisch herausgestellt, daß wiederum keine vollständige Ausnutzung der Saugfähigkeit des Tampons erreicht wird.

In der WO 94/15564 wird ein Verfahren zur Herstellung eines Tampons mit einer Kuppe mit einem konvexen Scheitelbereich offenbart, wobei der Tampon vollständig mit einer Umhüllung versehen wird. Die Befestigung des Rückholfadens erfolgt dabei nach dem Wickelvorgang durch Befestigung des Rückholfadens durch eine Öffnung quer zur Längsachse des Tampons und ist damit aufwendig.

Angesichts der vorstehend beschriebenen Probleme im Stand der Technik liegt der Erfindung die Aufgabe zugrunde, ein einfach ausführbares Verfahren bereitzustellen, mit dem Tampons erhalten werden, welche unter besonders guter Ausnutzung der Saugfähigkeit verwendet werden können, ohne daß die Gefahr des Ausfusselns besteht, und einen entsprechenden Tampon bereitzustellen. Weitere Verfahren zum Herstellen von Tampons sind angegeben in der US 8,827,974 B2, US 2003/0135180 A1, US 4,816100 und US 2012/0283684 A1,

Erfindungsgemäß wird diese Aufgabe durch die in Patentanspruch 1 angegebene Weiterbildung der bekannten Verfahren zum Herstellen eines eine Kuppe mit einem konvexen Scheitelbereich aufweisenden Tampons gelöst.

Die Erfindung geht auf die Erkenntnis zurück, daß die nur mangelhafte Ausnutzung der Saugfähigkeit von Tampons gemäß der EP 2 740 448 A1 im Wesentlichen darauf zurückzuführen ist, daß durch die Abdeckung der Stirnfläche eines Tampons vor der Verpressung und Bildung einer muldenartigen Vertiefung im Bereich der Tamponkuppe im Rahmen der Verpressung bzw. Kuppenformung, wie es in der EP 2 740 448 A1 vorgeschlagen wird, eine Hüllmaterialansammlung im Bereich der Vertiefung der Kuppe erfolgt, die ein Eindringen von Körperflüssigkeit über die Vertiefung in den Saugkörper behindert und so in einer nur mangelhaften Ausnutzung der Saugfähigkeit des Saugkörpers resultiert.

Im Rahmen der Erfindung wurde nun erkannt, daß eine optimale Ausnutzung der Saugfähigkeit des Saugkörpers bei gleichzeitiger Verhinderung des Ausfusselns durch eine Weiterbildung von beispielsweise in der DE 35 19 515 A1 beschriebenen Verfahren möglich wird, bei dem auch die Kuppe mit einem flüssigkeitsdurchlässigen Hüllmaterial abgedeckt wird, wobei eine Behinderung durch Ansammlung von Hüllmaterial im Bereich der Kuppe durch Vermeidung der Bildung einer Vertiefung in diesem Bereich ausgeschlossen wird. Als zusätzlicher Vorteil erfindungsgemäßer Tampons ist hervorzuheben, daß diese Tampons über eine besonders glatte Kuppe verfügen, weil diese insgesamt mit einem Hüllmaterial abgedeckt ist und so einfach in die Körperhöhle eingeführt werden können.

Ähnlich wie bei dem in der EP 2 740 448 A1 beschriebenen Verfahren wird auch bei dem erfindungsgemäßen Verfahren zweckmäßigerweise das Hüllmaterial vor dem Wickelvorgang auf der Materialbahn angebracht, insbesondere aufgesiegelt, wobei sich das Hüllmaterial vorzugsweise über die gesamte Breite der Materialbahn, aber nur über einen Teil der Länge davon erstreckt und an einem Längsende einzelner zur Bildung der Tampons auf vorgegebene Längen geschnittener Materialbahnabschnitte darüber hinausragt.

Wie bei den bekannten Verfahren zum Herstellen von Tampons wird die Materialbahn zum Erhalt von Bahnabschnitten vorgegebener Länge zur Herstellung jeweils eines Tampons vorzugsweise vor Aufbringen des Hüllmaterials längs senkrecht zu den Längsrändern verlaufenden Schnittlinien geschnitten und das Hüllmaterial unter Bildung eines derart über die etwa senkrecht zum Längsrand verlaufende Schnittlinie überstehenden Querüberstands auf den Bahnabschnitt aufgebracht, daß es nach dem Wickelvorgang eine Außenschicht des Wickels mit auf dem Hüllmaterial aufliegendem Querüberstand bildet. Der Querüberstand kann dann auf die darunterliegende Hüllmaterialschicht aufgesiegelt werden, um so eine stabile Umhüllung des Saugmaterials zu erhalten.

Erfindungsgemäß wird die Materialbahn vor dem Wickelvorgang mit einem Rückholfaden ausgestattet, der die Materialbahn umschlingt. Die freien Enden des die Materialbahn umschlingenden Rückholfadens werden dann mit Hilfe eines von der mit dem Rückholfaden ausgestatteten Materialbahn durchlaufenen Knoterapparats miteinander verknotet. Der Rückholfaden ist üblicherweise längs einem den Kern des Tampons bildenden vorderen und nicht mit Hüllmaterial abgedeckten Bereich der Materialbahn angeordnet.

Ähnlich wie bei bekannten Verfahren zum Herstellen von Tampons wird die Kuppe auch bei der Herstellung erfindungsgemäßer Tampons nach Verpressung des Wickels mit einem eine Kuppenformeinrichtung aufweisenden Formwerkzeug gebildet. Dabei wird bei der Herstellung erfindungsgemäßer Tampons durch geeignete Einstellung der Betriebsparameter der iks dafür gesorgt, daß das zuvor auf das saugfähige Material aufgesiegelte Hüllmaterial die Kuppe mit konvexem Scheitelbereich im Wesentlichen vollständig abdeckt, ohne bei der Formgebung Falten zu werfen und/oder lokale Materialansammlungen zu bilden.

Durch geeignete Temperaturführung kann insbesondere auch vermieden werden, dass das Hüllmaterial im Bereich der Kuppe aufgeschmolzen und so mit sich selbst verschweißt wird. Erfindungsgemäß ist das Hüllmaterial als Vlies, besonders bevorzugt thermobondiertes Krempelvlies ausgeführt. Es kann Bikomponentenfaser (100% Polyester/Polyethylen) enthalten oder daraus hergestellt sein.

Erfindungsgemäß wird die Temperatur der Kuppenformeinrichtung auf einen Wert von weniger als 160° C, vorzugsweise im Bereich zwischen 143° und 155° C eingestellt, um so eine Abdeckung der Kuppe mit dem Hüllmaterial ohne lokale Aufsohmelzungen bzw. Verschweißungen einzelner Hüllmateriallagen zu verwirklichen. Es hat sich gezeigt, dass die Reduzierung der Temperatur der Kuppenformeinrichtung im Vergleich zu der herkömmlicherweise auf 170° bis 190° C eingestellten Temperatur keine negativen Einflüsse auf das fertige Produkt hat.

Bei der Wahl des Hüllmaterials muss dafür gesorgt werden, daß die Abdeckung der Kuppe durch das 10 Hüllmaterial mit Hilfe der Kuppenformeinrichtung nicht zu einer Beschädigung des Hüllmaterials führt. In diesem Zusammenhang hat es sich als besonders günstig erwiesen, wenn das Hüllmaterial ein Vlies mit einem Flächengewicht von 4 g/m² oder mehr, vorzugsweise 12 g/m² oder mehr aufweist. Ein Hüllmaterial mit einem geringeren Flächengewicht neigt dazu, während des Formgebungsvorgangs beschädigt zu werden. Andererseits muss darauf geachtet werden, daß das Hüllmaterial bei der Ausformung der Kuppe keine Verwerfungen bildet, welche den Tragekomfort und/oder den Einführvorgang beeinträchtigen. In diesem Zusammenhang hat es sich als günstig erwiesen, wenn das Flächengewicht des vliesförmigen Hüllmaterials 50 g/m² oder weniger, insbesondere 40 g/m² oder weniger beträgt. Eine Beschädigung des Hüllmaterials während des Formgebungsvorgangs kann besonders zuverlässig ausgeschlossen werden, wenn die Reißkraft des Hüllmaterials in Längsrichtung 3 N/inch oder mehr, insbesondere 8 N/inch oder mehr beträgt, wobei ein Wert von 9,5 N/inch besonders bevorzugt ist, und/oder die Bruchdehnung des Hüllmaterials 10 Prozent oder mehr, insbesondere 20 Prozent oder mehr, besonders bevorzugt 25 Prozent beträgt. Im Hinblick auf einen ausgewogenen Kompromiss zwischen Kosten auf der einen Seite und der gewünschten mechanischen Eigenschaften auf der anderen Seite hat es sich als zweckmäßig erwiesen, wenn die Bruchdehnung des Hüllmaterials 50 Prozent oder weniger, vorzugsweise 30 Prozent oder weniger beträgt. In jedem Fall ist die optimale Reißkraft und Bruchdehnung des Hüllmaterials auch vom Flächengewicht des Hüllmaterials abhängig. Bei einem höheren Flächengewicht ergibt sich eine größere Reißkraft, was aber auch automatisch mit höheren Kosten verbunden ist. Aus diesem Grund wird das Flächengewicht zweckmäßigerweise auf die oben angegebenen Werte eingestellt, wobei sich die angegebenen optimalen Werte für Reißkraft und Bruchdehnung ergeben. Sofern eine höhere Reißkraft für besondere Anwendungen gewünscht ist, kann dies durch entsprechende Erhöhung des Flächengewichts sichergestellt werden.

Eine Vorrichtung zum Herstellen erfindungsgemäßer Tampons weist im Wesentlichen eine Wickeleinrichtung zum Herstellen eines Wickels aus einer Materialbahn aus saugfähigem Material und einer Presseinrichtung zum Verpressen des Wickels sowie eine Kuppenformeinrichtung zur Bildung der den konvexen Scheitelbereich aufweisenden Kuppe auf und ist im Wesentlichen dadurch gekennzeichnet, daß sie mit einer Abdeckeinrichtung zum Abdecken einer Stirnfläche des Wickels mit dem Hüllmaterial ausgestattet ist. Dabei kann die Vorrichtung eine Zuführeinrichtung zum Aufbringen des Hüllmaterials auf die Materialbahn aufweisen. Die Wickeleinrichtung kann eine Wickelhülse und einen in der Wickelhülse bezüglich der Wickelachse drehbar gelagerten Wickeldorn umfassen, wobei sowohl die Wickelhülse als auch der Wickeldorn einen sich in Richtung auf die Hülsenachse erstreckenden Aufnahmeschlitz aufweisen können, in den die Materialbahn mit dem darauf aufgebrachten Hüllmaterialband einführbar ist, wobei sich der Innendurchmesser der Wickelhülse in Richtung auf ein offenes Ende davon verjüngt und/oder der Außendurchmesser des Wickeldorns sich in Richtung auf dieses Ende vergrößern kann.

Im Rahmen der Erfindung ist es von besonderer Bedeutung, daß das Hüllmaterial derart auf die Materialbahn aufgebracht werden kann, daß es sich über die gesamte Breite der Materialbahn erstreckt, um so im weiteren Verlauf der Tamponherstellung die den konvexen Scheitelbereich aufweisende Kuppe zumindest teilweise, insbesondere im Wesentlichen vollständig, abdecken zu können. Das Hüllmaterial kann auch so konfektioniert sein, dass es geringfügig über die Materialbahn, auf die es aufgebracht wird, hinausragt. In einigen Fällen kann es vorteilhaft sein, wenn das Hüllmaterial geringfügig schmaler ist als die Materialbahn.

Im Hinblick auf weitere Details erfindungsgemäßer Vorrichtungen und Herstellungsverfahren wird auf entsprechende Erläuterungen in der EP 2 740 448 A1, der EP 2 298 259 A1 und EP 2 431 014 A1 Bezug genommen, deren Offenbarungsgehalt hinsichtlich der Ausführung von Vorrichtungen zum Herstellen von Tampons und entsprechenden Herstellungsverfahren hiermit durch ausdrückliche Inbezugnahme in diese Beschreibung aufgenommen wird.

## Patentansprüche

1. Verfahren zum Herstellen eines in die weibliche Scheide einführbaren Tampons mit einem zylinderförmigen Saugkörper, der eine sich in Richtung auf ein axiales Ende des Saugkörpers verjüngende Kuppe mit einem konvexen Scheitelbereich aufweist, wobei der Ausdruck "konvexer Scheitelbereich" eine Oberflächenform bezeichnet, bei der jeder einzelne Punkt auf der Oberfläche der Kuppe mit Hilfe von innerhalb der Kuppe verlaufenden Linien mit jedem anderen Punkt auf der Oberfläche der Kuppe verbunden werden kann, bei dem eine Materialbahn aus saugfähigem Material zu einem Wickel aufgewickelt und der Wickel zumindest abschnittweise in radialer und/oder axialer Richtung verpreßt wird, bei dem die Kuppe mit einem flüssigkeitsdurchlässigen Hüllmaterial abgedeckt wird, **dadurch gekennzeichnet, dass** vor dem Wickelvorgang ein Rückholfaden um die Materialbahn geschlungen wird und die von dem Rückholfaden umschlungene Materialbahn einen Knoterapparat durchläuft, wobei die Kuppe mit einer Kuppenformeinrichtung gebildet wird, das Hüllmaterial als Vlies ausgeführt ist und Polyester/Polyethylen-Bikomponentenfasern enthält und die Temperatur der Kuppenformeinrichtung auf einen Wert von weniger als 160° C eingestellt wird, um so eine Abdeckung der Kuppe mit dem Hüllmaterial ohne lokale Aufschmelzungen bzw. Verschweißung einzelner Hüllmateriallagen zu verwirklichen.

2. Verfahren nach Anspruch 1, **dadurch gekennzeichnet, daß** das Hüllmaterial vor dem Wickelvorgang auf die Materialbahn aufgebracht, insbesondere aufgesiegelt wird, wobei sich das Hüllmaterial etwa über die gesamte Breite der Materialbahn erstreckt oder darüber hinausragt.

3. Verfahren nach Anspruch 1 oder 2, **dadurch gekennzeichnet, daß** die Materialbahn zu Bahnabschnitten vorgegebener Länge geschnitten und das Hüllmaterial unter Bildung eines Querüberstands derart über eine etwa senkrecht zum Längsrand der Materialbahn verlaufende Schnittlinie auf den Bahnabschnitt aufgebracht wird, daß das Hüllmaterial nach dem Wickelvorgang eine Außenschicht des Wickels mit auf dem Hüllmaterial aufliegendem Querüberstand bildet.

4. Verfahren nach einem der vorhergehenden Ansprüche, **dadurch gekennzeichnet, daß** nach Verpressen des Wickels die Kuppe mit einem Formwerkzeug einer Kuppenformeinrichtung derart gebildet wird, daß sie von dem Hüllmaterial abgedeckt ist.

5. Verfahren nach einem der vorhergehenden Ansprüche, **dadurch gekennzeichnet, daß** das Hüllmaterial ein Vlies mit einem Flächengewicht von 4 g/m² oder mehr und/oder weniger als 50 g/m² aufweist.

## Claims

1. A method of producing a tampon that can be introduced into a woman's vagina comprising a cylindrical absorbent body which has a summit with a convex crown area that tapers towards an axial end of the absorbent body, wherein "convex crown area" designates a surface shape where each single point on the surface of the summit can be connected with each other point on the surface of the summit by means of lines running within the summit, a material web of absorbent material being wound into a winding and the winding being pressed at least in sections in the radial and/or axial direction, the summit being covered with a liquid-permeable covering material, **characterized in that,** before the winding process, a retrieval string is looped around the material web and the material web around which the retrieval string is looped runs through a knotting apparatus, wherein a summit moulding device is used to form the summit, the covering material is made of a non-woven and contains polyester/polyethylene bicomponent fibres, and the temperature of the summit moulding device is set to a value of less than 160°C in order to achieve a covering of the summit with the covering material without localised melting on or welding together of individual layers of covering material.

2. The method according to claim 1, **characterized in that** the covering material is applied, in particular sealed over, the material web, the covering material spreading over or extending beyond the entire width of the material web.

3. The method according to claims 1 or 2, **characterized in that** the material web is cut into web sections of defined length and the covering material is applied to the web section such as to form a transverse projection over the section line running approximately perpendicular to the longitudinal edge such that the covering material forms an outer layer of the winding with a transverse projection lying over the covering material after the winding process.

4. The method according to any of the preceding claims, **characterized in that** after pressing the winding the summit is formed by a moulding tool of a summit moulding device such that it is covered by the covering material.

5. The method according to any of the preceding claims, **characterized in that** the covering material features a non-woven with a surface weight of 4 g/m² or more and/or less than 50 g/m².

## Revendications

1. Procédé de fabrication d'un tampon susceptible d'être introduit dans le vagin, comprenant un corps absorbant cylindrique qui présente une extrémité arrondie qui va en rétrécissant en direction d'une extrémité axiale du corps absorbant, l'extrémité arrondie étant pourvue d'un sommet convexe, l'expression « sommet convexe » désignant une forme de surface selon laquelle chaque point individuel présent sur la surface de l'extrémité arrondie peut être relié, par des lignes parcourant l'extrémité arrondie, à chaque autre point présent sur la surface de l'extrémité arrondie ; dans lequel une bande de matière absorbante est enroulée pour former un enroulement et l'enroulement est comprimé au moins par sections dans le sens radial et/ou axial, et l'extrémité arrondie est recouverte d'un matériau d'enveloppe perméable aux liquides, **caractérisé en ce que,** avant le processus d'enroulement, un fil de retrait est passé autour de la bande de matière et la bande de matière entourée du fil de retrait traverse un appareil noueur, ladite extrémité arrondie étant formée par un dispositif de façonnage d'extrémité arrondie, ledit matériau d'enveloppe étant conçu sous la forme d'un matériau non tissé et contenant des fibres bicomposants de polyester/polyéthylène, et la température du dispositif de façonnage d'extrémité arrondie étant réglée à une valeur inferieure à 160 °C afin de mettre en œuvre un recouvrement de l'extrémité arrondie par ledit matériau d'enveloppe sans fusions locales ou soudure de couches de matériau d'enveloppe individuelles.

2. Procédé selon la revendication 1, **caractérisé en ce que** le matériau d'enveloppe est appliqué, ou plus spécifiquement scellé, sur la bande de matière avant le processus d'enroulement, ledit matériau d'enveloppe s'étendant sur toute la larg[M1]eur de la bande de matière ou dépassant de la bande de matière.

3. Procédé selon la revendication 1 ou 2, **caractérisé en ce que** la bande de matière est découpée en sections de bande de longueur prédéfinie et le matériau d'enveloppe est appliqué sur la section de bande en formant un excédent transversal, le long d'une ligne de découpe orientée de manière sensiblement perpendiculaire au bord longitudinal de la bande de matière de telle manière que, suite au processus d'enroulement, le matériau d'enveloppe forme une couche extérieure de l'enroulement qui présente un excédent transversal reposant sur le matériau d'enveloppe.

4. Procédé selon l'une quelconque des revendications précédentes, **caractérisé en ce que,** après la compression de l'enroulement, l'extrémité arrondie est formée par un outil de façonnage d'un dispositif de façonnage d'extrémité arrondie, de telle manière que l'extrémité arrondie se retrouve recouverte du matériau d'enveloppe.

5. Procédé selon l'une quelconque des revendications précédentes, **caractérisé en ce que** le matériau d'enveloppe présente un matériau non tissé ayant un grammage de 4 g/m² ou plus et/ou 50 g/m² ou moins.
